# EUROPEAN PATENT SPECIFICATION

(11) **EP 1 393 637 B1**
(45) Date of publication and mention of the grant of the patent: **14.11.2007**
(21) Application number: 02769566.7
(22) Date of filing: 10.05.2002
(51) Int. Cl.: A23L 1/30, A23G 3/00, A23G 4/10, A23L 1/23, A23L 2/60

(54) **USE OF PALATINOSE FOR SUSTAINING MENTAL CONCENTRATION AND ATTENTIVENESS**
VERWENDUNG VON PALATINOSE ZUR AUFRECHTERHALTUNG DER MENTALEN KONZENTRATIONSFÄHIGKEIT UND AUFMERKSAMKEIT
UTILISATION DE PALATINOSE PERMETTANT DE MAINTENIR LES CONCENTRATION ET ATTENTION MENTALES

(30) Priority: 14.05.2001 JP 2001143370; 24.07.2001 JP 2001223004
(43) Date of publication of application: 03.03.2004
(73) Proprietor: Mitsui Sugar Co., Ltd., Chuo-ku, Tokyo (JP)
(72) Inventor: KASHIMURA, Jun, Ota-ku, Tokyo 144-0054 (JP); MIZUTANI, Takeo, Yokohama-shi, Kanagawa 221-0863 (JP); WATANABE, Ichiro, Meguro-ku, Tokyo 153-0042 (JP)
(74) Representative: Bawden, Peter Charles
(86) International application number: PCT/JP2002/004578
(87) International publication number: WO 2002/091859

(56) References cited:
- WO-A1-91/15941
- JP-A- 57 058 852
- JP-A- 60 102 144
- US-A- 4 572 916
- US-A- 5 292 536
- PATENT ABSTRACTS OF JAPAN vol. 2000, no. 12, 3 January 2001 (2001-01-03) & JP 2000 245390 A (EZAKI GLICO CO LTD), 12 September 2000 (2000-09-12)

## Description

### BACKGROUND OF THE INVENTION

### FIELD OF THE INVENTION

This invention relates to an agent comprising palatinose for sustaining mental concentration and attention, and a food and a drink comprising the agent. Particularly, this invention relates to an agent for enhancing mental concentration and attention and also for maintaining mental concentration and attention over a longer time and more constantly, and a food and a drink comprising the agent.

### DESCRIPTION OF THE PRIOR ART

It is known that comprehension and ability of getting an idea are increased by enhancing concentration and attention. Therefore, it was studied in various aspects how to enhance concentration and attention.

It is said that a state, "concentrating", is a mental condition where a person is moderately relaxed and moderately tense. It is also said that concentration is roughly divided in two categories, instantaneous concentration and sustained concentration. The instantaneous concentration is ability exerted in a moment of time, which is realized by bringing one's ability into full play, for example, ability of getting through a sudden crisis, ability of getting an idea, and ability of judgment and handling in sports. The sustained concentration is ability of continuing a monotonous work for a long time, or dealing with a single long-term work with patience to finish it to the end, for example, ability for simple calculation jobs, ability for short-term memory, attention in driving a car, and continued learning ability to prepare for an examination.

To enhance concentration, many methods are known, such as a method by food, a method by aroma such as aromatherapy and incense, a method by refreshment of mind and body, a self-control method, a method by enhancing one's volition, a method by getting rid of stress, a method by concentrating attention on one point, a method by putting environment in order, and a breathing method by Zen sitting meditation.

As the methods of enhancing concentration by taking food, the following is known.

Balance between moderate tension and moderate relaxation, which is necessary for concentration, is created by a combination of herb or lemon grass, which is supposed to enhancemental tension, with calcium which is supposed to suppress irritation, stabilize mind and create relaxation. As a result, concentration is enhanced.

As another method, it is also known that concentration is improved by taking sucrose. Although the mechanism is not clear, is supposed that one reason for it may be elevation of the blood sugar level.

In the modern society, people are, regardless of age and sex, very often required to have concentration and attention, especially for examinations and jobs. If concentration and attention can be enhanced and maintained over a longer time and more constantly by taking a particular food, this is very advantageous in examinations and jobs. Thus, it is desired to solve the aforesaid problems by taking an edible material.

### SUMMARY OF THE INVENTION

The present inventors have found that mental concentration and attention can be enhanced and sustained over a longer time and more constantly by taking palatinose.

Thus, the present invention provides an agent for sustaining mental concentration and attention, which comprises palatinose.

The present invention also provides a food and a drink comprising the agent.

### BRIEF DESCRIPTION OF THE DRAWINGS

Fig. 1 is a graph which shows the results of a Kraepelin test on a mixture of aspartame and acesulfame potassium as a control.
Fig. 2 is a graph which shows the results of a Kraepelin test on a test group and a comparative group in Example 1.
Fig. 3 is a graph which shows the results of a sequential memory test on the test group and the comparative group in Example 2.

### DESCRIPTION OF THE PREFERRED EMBODIMENTS

Palatinose, which is also called isomaltulose, is a disaccharide in which glucose is bonded to fructose via α-1,6-glucosyl bonding. The properties of palatinose monohydrate crystal are as follows: melting point, 123 - 124 C; specific rotation, [α] ²⁰_{D}=+97.2; Fehl ing' s solution reducing power, 52 % of glucose; solubility in 100 g of water, 38.4 g at 20 C, 78.2 g at 40 C, and 174.9g at 60 C; quality of sweet taste of its aqueous solution, good; and the degree of sweetness, about 42 % of sucrose.

Palatinose is found in honey and cane juice in nature. It is also present in a rearrangement reaction product which is formed when α-glucosyltransferase originating from bacteria or yeast acts on sucrose.

Commercially, sucrose is converted mostly into palatinose by subjecting sucrose to the action of α-glucosyltransferase which is produced by bacteria, such as Protaminobacter rubrum or Serratia plymuthica.

In the present specification, the terms "concentration and attention" refer to the mental ability to mainly do consideration relating to the following works and to increase the working efficiency when a person does works which require consideration such as getting an idea, judgment, calculation, and memory. The works which require consideration include simple calculation works, recall of short-term memory, driving an automobile, learning work for examination, association of ideas game, plays on words such as crossword puzzle and shiritori (or verses-capping game), games using brain such as game of go or shougi (Japanese chess) game, and sports which require game plans such as ball handling or time allotment.

The agent for sustaining concentration and attention in the present invention includes crystalline palatinose, and palatinose syrup. Crystalline palatinose available on the market (Crystalline Palatinose-IC, trade name, ShinMitsui Sugar Co., Ltd.) contains 99.0 % or more of palatinose including crystal water). Palatinose syrup available on the market (Palatinose Syrup-ISN or -TN, trade name, Shin Mitsui Sugar Co., Ltd.) contains 11 - 17 % of palatinose and, further, trehalulose. Trehalulose syrup available on the market (Mildear-75 or 85, trade name, Shin Mitsui Sugar Co., Ltd.) contains 8 - 13 % of palatinose and, therefore, may be used as the present agent.

Alternatively, the agent for sustaining concentration and attention may be fondants, granules, tablets, powder mixtures (which comprise an acidulant, sweetener, sugar ester, flavor etc.) orsyrups, comprising crystalline palatinose or palatinose syrup.

Typically, palatinose is taken in an amount of 5 to 50 g at once, preferably 5 to 20 g.

The food and the drink comprising the agent for sustaining concentration and attention according to the present invention include various types of beverage, such as soft drinks, near water, sports drinks , jelly drinks, tea drinks, cocoa drinks and coffee drinks, and various types of foods, such as soft jellies, hard candies, soft candies, chocolates and chewing gums. Alternatively the agent for sustaining concentration and attention according to the present invention may be added as a sweetener in coffee, tea or cocoa.

By taking the agent for sustaining concentration and attention or a food or drink comprising the agent according to the present invention, concentration and attention are enhanced and maintained over a longer time and more constantly.

The Kraepelin test is originally a psychological test to know features in jobs and actions of a subject where the subject continues addition of two numerals of a single digit for 30 minutes. Recently, the Kraepelin test is often used as a method for determining concentration and attention. Specifically, in the present test, a subject makes addition on numerals in a first line from the left to the right for one minute (1st round) , and then do the same on the second line (2nd round) . This is repeated on total 15 lines for 15 minutes.

Immediately before drinking a test sample, a subject takes the aforesaid test, and an average score for 15 lines is determined. Then, the subj ect takes a test sample and, after a predetermined time, the subject takes the aforesaid test again and an average score is calculated. The second average minus the first average reflects the effect of the test sample on the sustention of concentration and attention.

To obtain more reliable results, plural subjects take the test and the average score of the plurality of subjects is evaluated. It is recommendable to do a t-test for paired samples. In judgment in the t-test, a significant difference is usually recognized at 5 % or less of a risk level (p < 0.05), or 1 % or less of a risk level (p < 0.01).

When the Kraepelin test is performed on materials which have sweet taste, it is desirable that the degree of sweetness is equal among them. If the degree of sweetness is not equal with each other, it might happen that the score of a group who took a higher degree of the sweetness is higher than that of a group who took a lower degree of the sweetness, because of a psychological effect.

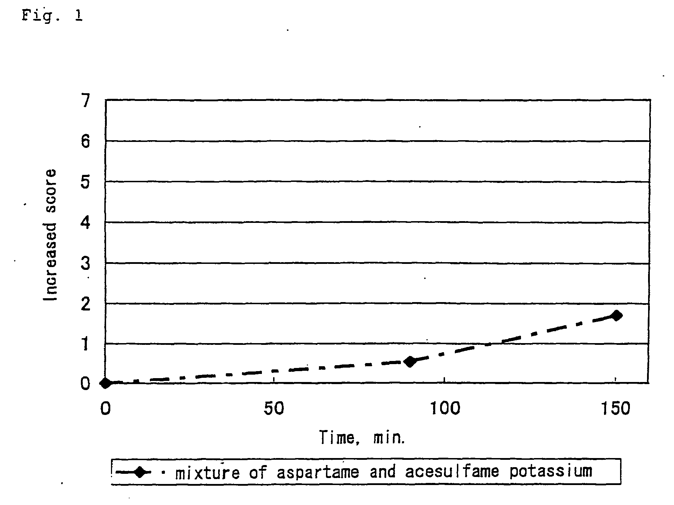

As mentioned above, the degree of sweetness of palatinose is 42, with the degree of sweetness of sucrose being 100. Accordingly, aspartame and acesulfame potassium are added to a palatinose sample to adjust the degree of sweetness to that of a sucrose sample while the intake weight of palatinose is set equal to that of sucrose. As seen in the following preliminary test and in Fig. 1, the mixture of aspartame and acesulfame potassium scarcely raises the score in the Kraepelin test.

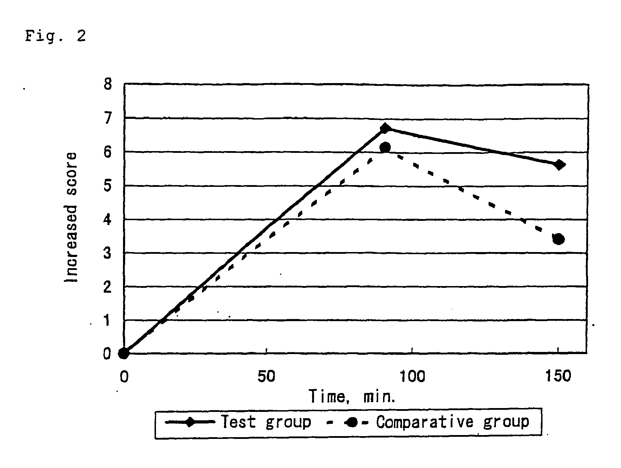

As shown in the following Example 1 and Fig. 2, it has been found that palatinose (for the test group) has the effect of sustaining concentration and attention in the Kraepelin test. The increase in the score is about 6.7 and about 5.6 at 90 minutes and 150 minutes after takingpalatinose, respectively. This shows that concentration and attention is constantly sustained by taking palatinose.

On the other hand, the increase in the score in the comparative group by taking sucrose is about 6.1 after 90 minutes. However, the increase in the score after 150 minutes is about 3.4, which is almost half as large as the score after 90 minutes.

In the t-test for paired samples, the difference between the average score after 90 minutes with palatinose (test group) and the initial average score is recognized as significant with 1 % or less of a risk level; and the difference after 150 minutes is also significant with 5 % or less of a risk level. For sucrose (comparative group), the difference after 90 minutes is recognized as significant with 1 % or less of a risk level, but the difference after 150minutes is recognizedas non-significant because of a risk level of 17 %.

From the above, it is concluded that palatinose gives a significantly increased score for a longer time period than sucrose does.

Another method, sequential memory test, is known to determine the sustainability of concentration and attention. In this test, a score of correct answers is regarded as ability for short-term memory. More specifically, several sheets of paper on which a meaningless series of katakana characters or several digits of numerals are written are shown to a subject for two seconds per sheet. Then, the subject writes what was written on each sheet orderly, in an answer sheet. A correct answer is given one point in score. This is repeated several times.

In the present invention, the sustainability of the concentration and attention is determined in the sequential memory test as follows. First, the sequential memory test is performed on a subject immediately before drinking a test sample to determine an initial score. Then, the test sample is taken and the sequential memory test is performed after a predetermined time. For the sequential memory test, a card on which five digits of numerals are written is shown to a subj ect for two seconds. Total five sheets are presented. Then, the subject writes all of the shown five digits of numerals in an answer form. This is repeated 5 times to make a set of tests. Therefore, perfect answer is given 25 points. The sustainability of concentration and attention is judged by whether the score after the predetermined period of time is significantly, increased, compared to the initial score.

As mentioned for the Kraepelin test on materials which have sweet taste, it is desirable that the degree of sweetness is equal among them. Therefore, as in the Kraepelin test, aspartame and acesulfame potassium are added to a palatinose sample to adjust the degree of sweetness to that of a sucrose sample, while the intake weight of palatinose is set equal to that of sucrose.

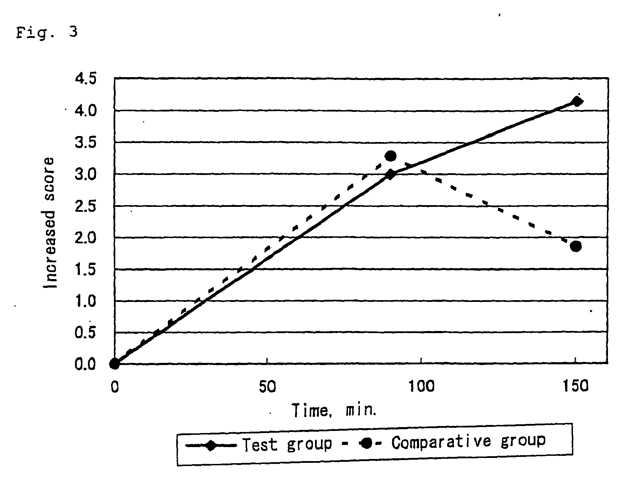

As shown in the following Example 2 and Fig. 3, it has been found that palatinose (for the test group) has the effect of sustaining concentration and attention in the sequential memory test. The increase in the score is about 3.0 and about 4.1 at 90 minutes and 150 minutes after taking palatinose, respectively. On the other hand, the increase with sucrose (for the comparative group) is about 3.3 and about 1.9, respectively. That is, the increase in score of palatinose is a little lower than that of sucrose at 90 minutes, but is more than two times as high as that of sucrose at 150 minutes.

In the t-test for paired samples, the differences between the average score after 90 minutes and 150 minutes with palatinose (for the test group) and the initial average score are both recognized as significant with 1 % or less of a risk level. For sucrose (for the comparative group), the increases in score after 90 minutes are various among the subjects, but the difference is recognized as significant with 5 % or less of a risk level. The difference after 150 minutes is recognized as significant with 5 % or less of a risk level.

From the above, it is concluded that palatinose gives a significantly increased score for a longer time period than sucrose does.

Judging from the aforesaid results of the Kraepelin test and the sequential memory test, concentration and attention can be raised in a short term by taking sucrose, but cannot sustain concentration and attention over a long time and constantly.

On the other hand, it is clear that palatinose can sustain concentration and attention over a longer time and more constantly than sucrose does.

The invention will be explained in more detail with reference to the Examples, but the invention shall not be limited by these Examples.

### EXAMPLES

### Control test

### 1) Test procedures

Seven healthy six males and one female aged 24 to 59 years were chosen as subjects. The subjects did not take breakfast on the test day to be in abstinence from diet for 12 hours or more before starting the test.

First, the Kraepelin test was performed for 15 minutes (15 rounds) without taking any food or drink to determine an initial score. In the next 5 minutes, the subjects took 200mL of a drink in which 0.12 g of aspartame and 0.12 g of acesulfame potassium were dissolved in 200mL of distilled water.

The Kraepelin test was performed for 15 minutes (15 rounds) at 90 minutes and 150 minutes after taking the drink, and the average increase in score of the subjects was calculated.

### 2) Test results

The results are as shown in Fig. 1. The mixture of aspartame and acesulfame potassium scarcely increased the score in the Kraepelin test. It is noted that the score increased in the test after 150 minutes, but this is presumably because the subjects were accustomed to the Kraepelin test.

### Example 1

40g of crystalline palatinose (Crystal Palatinose-IC, trade name, Shin Mitsui Sugar Co., Ltd.), 0.06 g of aspartame and 0.06 g of acesulfame potassium were dissolved in 200mL of distilled water to prepare a drink containing the agent of the present invention (hereinafter, referred to as "the drink of Example 1"). The amounts above are for the use by one subject

The addition of these amounts of aspartame and acesulfame potassium to 40 g of palatinose in 200 mL of distilled water gives the same degree of sweetness as that of 40 g of sucrose in 200 mL of distilled water.

### 1) Test procedures

Healthy twelve males and two females aged 25 to 59 years who were different from those in the control test were chosen as subjects, and they were divided into two groups with each seven persons (a test group and a comparative group) so that the average ages of the two groups were even.

The subjects did not take breakfast on the test day to be in abstinence from diet for 12 hours or more before starting the test.

First, the Kraepelin test was performed for 15 minutes (15 rounds) without taking any food or drink to determine an initial score. In the next 5 minutes, the test group took 200mL of the drink of Example 1 and the comparative group took 200mL of a drink in which 40g of sucrose was dissolved in 200mL of distilled water.

The Kraepelin test was performed for 15 minutes at 90 minutes and 150 minutes after taking the drink, and the average increase in score of the subjects in each group was calculated. The t-test for paired samples was made.

### 2) Test results

The results are as shown in Fig. 2. At 90 minutes after taking the drinks, the increase in score was about 6.7 in the test group; and about 6.1 in the comparative group.

At 150 minutes after taking the drinks, the increase in score is about 5.6 in the test group; and about 3.4 in the comparative group. This shows that the test group indicated a clearly better result than the comparative group at 150 minutes after taking the drinks.

In the test group, the increase in score is about 6.7 at 90 minutes and about 5.6 at 150 minutes, so that the fall in score with time is little . On the other hand, in the comparative group, the increase in score is about 6.1 at 90 minutes and about 3.4 at 150 minutes, so that the fall in score with time is apparent.

The t-test for paired samples was conducted on the difference between the score at 90 minutes and the initial score and on the difference between the score at 150 minutes and the initial score. For the teat group, the difference between the average score at 90 minutes and the initial score is recognized as significant with 1 % or less of a risk level; and the difference at 150 minutes, significant with 5 % or less of a risk level. For the comparative group, the difference for 90 minutes is recognized as significant with 1 % or less of a risk level, but the difference for 150 minutes is recognized as non-significant because of a risk level of 17 %.

From the above, it is seen that palatinose significantly increases the score and maintains the significant increase over a long time and constantly.

### Example 2

### 1) Test procedures

Healthy twelve males and two females aged 25 to 59 years who were different from those in the control test were chosen as subjects, and they were divided into two groups with each seven persons (a test group and a comparative group) so that the average ages of the two groups were even.

The subjects did not take breakfast on the test day to be in abstinence from diet for 12 hours or more before starting the test.

First, the sequential memory test was performed five times with a set of five cards indicating a 5-digit numeral thereon, without taking any food or drink to determine an initial score. In the next 5 minutes, the test group took 200mL of the drink of Example 1 and the comparative group took 200mL of a drink in which 40g of sucrose was dissolved in 200mL of distilled water.

The sequential memory test was performed at 90 minutes and 150 minutes after taking the drink, and the average increase in score of the subjects in each group was calculated.

### 2) Test results

The results are as shown in Fig. 3. In the test group, the increase in score is about 3.0 at 90 minutes after taking the drinks; and about 1. 9 at 150 minutes. The t-test for paired samples was conducted on the di f ference at 90 minutes and on the difference at 150 minutes in comparison with the initial score to find that the differences are both significant with a risk level of 1 % or less.

In the comparative group, the increase in score is about 3.3 at 90 minutes after taking the drinks; and about 1. 9 at 150 minutes The t-test for paired samples was conducted on the difference at 90 minutes and on the difference at 150 minutes to find that the differences are both significant with a risk level of 5 % or less.

From above, a significant difference is recognized between the score at 90 minutes and the initial score and between the score at 150 minutes and the initial score for both the test group and the comparative group. However, judging from the risk levels, the test group maintains more stable and more significant increase in score over a longer time than the comparative group does.

### Example 3

An agent according to the present invention, fondant, was manufactured in the following recipe. Crystalline palatinose (Crystal Palatinose-IC, trade name of Shin Mitsui Sugar Co., Ltd.) was fed at a raw material feeder of a twin-screw extruder at a rate of 120 kg per hour, and melted at 160 - 200 C. After this location, water was injected for cooling at a rate of 5.6 kg per hour to prepare microcrystals . Finally, palatinose syrup (Palatinose Syrup-ISN, trade name of ShinMitsui Sugar Co., Ltd.) was injected at a rate of 100 kg per hour and mixed under cooling.

### Example 4

An agent according to the present invention, granules, was manufactured using crystalline palatinose powder. Crystalline palatinose (Crystal Palatinose-IC, trade name of Shin Mitsui Sugar Co., Ltd., 70 % or more of 28 - 68 mesh powder) was fed to a raw material feeder of a twine-screw extruder at a rate of 40kg per hour, and melted at 160 - 180 C. After this location, water was added for cooling and precipitation at a rate of 2 kg per hour to obtain powder. The powder was sieved to collect granules, 99 % or more of which was of 10 - 40 mesh.

### Example 5

An agent according to the present invention, tablets, was manufactured in the following recipe. Mixed powder of the following recipe was compressed at a tablet pressure of 300 kg per cm² to form tablets which each have a diameter of 18 mm, thickness of 5 mm, and weight of 1.5 g.

| | |
|---|---|
| Pulverized palatinose (prepared by pulverizing the agent manufactured in Example 4 with an atomizer crusher) | 55 parts by weight |
| Citric acid | 1 parts by weight |
| Sugar ester | 1 parts by weight |
| Aspartame | 0.05 part by weight |
| Vitamin P | 0.0002 part by weight |
| Water | 0.6 part by weight |
| Lemon flavor | proper amount |

### Example 6

An agent according to the present invention, powder mixture, was manufactured in the following recipe using a universal mixer in accordance with a conventional manner.

| | |
|---|---|
| Crystalline palatinose (Crystal Palatinose-IC) | 84.7 parts by weight |
| Powdered fruit juice | 10 parts by weight |
| Citric acid anhydride | 3 parts by weight |
| Sodium citrate | 0.4 part by weight |
| L-Ascorbic acid | 0.5 part by weight |
| Sodium ascorbate | 0.3 part by weight |
| Riboflavin, 10 wt.% content | 0.1 part by weight |

### Example 7

Sugar-coated tablets which contained the agent according to the present invention were manufactured using the tablets manufactured in Example 5. The tablets manufactured in Example 5 were put into a coating pan and soft-coated with a syrup of the following recipe (a) and with powder isomalt, alternately, at a weight ratio of 1:2 , and subsequently hard-coated with a syrup of the following recipe (b). After the coating, they were air-dried using air of atmospheric temperature.

### (a) For the soft coating

| | |
|---|---|
| Powder isomalt (Palatinit, type GS, trade name of Shin Mitsui Sugar Co., Ltd.) | 62 % by weight |
| Gum arabic | 6.5 % by weight |
| Water | 31.5 % by weight |

### (b) For the hard coating

| | |
|---|---|
| Powder isomalt (Palatinit, type GS) | 65 % by weight |
| Gum arabic | 3.5 % by weight |
| Water | 31.5 % by weight |
| Lemon flavor | proper amount. |

### Example 8

A drink containing the agent according to the present invention was manufactured by using the agent, powdermixture, manufactured in Example 6. Twenty five grams of the agent manufactured in Example 5 were dissolved in 200 ml of hot water.

### Example 9

Arefreshing drink containing the agent according to the present invention was manufactured in the following recipe. The following ingredients were dissolved in 250 ml of hot water and filled in a drink can for 250 mL.

| | |
|---|---|
| Crystalline palatinose (Crystal Palatinose-IC) | 8 parts by weight |
| Citric acid | 0.15 part by weight |
| Vitamin C | 0.03 part by weight |
| Sodium chloride | 0.05 part by weight |
| Potassium chloride | 0.04 part by weight |
| Calcium chloride | 0.012 part by weight |
| Magnesium carbonate | 0.002 part by weight |
| Sodium glutamate | 0.006 part by weight |
| Stevia | 0.01 part by weight |
| Vitamin P | 0.0004 part by weight |
| Flavour | proper amount. |

### Example 10

Hard candies containing the agent according to the present invention were manufactured in the following recipe. First, crystalline palatinose, trehalulose syrup and water were added to a dissolver, and heated to dissolve under stirring. Then, they were heated at the temperature of 120 C at a reduced pressure of 700 mmHg, and then citric acid, aspartame, vitamin P and lemon flavor were mixed. After cooled to about 70 - 80 C, the mixture was shaped into candies of each 4 grams, which were packaged each separately.

| | |
|---|---|
| Crystalline palatinose (Crystal Palatinose-IC) | 70 parts by weight |
| Trehalulose syrup (Mildear-75, Shin Mitsui Sugar Co., Ltd.) | 40 parts by weight |
| Citric acid | 2 parts by weight |
| Aspartame | 0.07 part by weight |
| Vitamin P | 0.003 part by weight |
| Water | 15 parts by weight |
| Lemon flavor | proper amount. |

### Example 11

Jelly drinks with orange taste containing the agent according to the present invention were manufactured in the following recipe. First, a palatinose syrup and water were mixed with each other, to which a gelling agent was added portionwise to dissolve while heated up to 90 C. Then, the mixture was cooled to 70 C, to which the remaining ingredients were added and mixed to dissolve. The solution was filled in cheer packs, sealed, and sterilized at a temperature of 90 C for 20 minutes, and then cooled.

| | |
|---|---|
| Palatinose syrup, Bx. 75 (Palatinose Syrup-TN, ShinMitsui Sugar Co., Ltd.) | 15 parts by weight |
| Gelling agent | 1 part by weight |
| Orange fruit juice, 1/5 concentrated | 4 parts by weight |
| Water | 80 parts by weight |
| Citric acid | 0.35 part by weight |
| Sodium citrate | 0.20 part by weight |
| Vitamin C | 0.6 part by weight |
| beta-Carotene | 0.01 part by weight |
| Stevia | 0.01 part by weight |
| Vitamin P | 0.0004 part by weight |
| Orange flavor | proper amount. |

### INDUSTRIAL APPLICABILITY

By taking the agent for sustaining concentration and attention according to the present invention, one can enhances his or her concentration and attention and also maintain his or her concentration and attention over a longer time and more constantly.

## Claims

1. The use of palatinose in the manufacture of an agent for sustaining mental concentration and attention.

2. The use according to claim 1, wherein the agent for sustaining concentration and attention is a food or a drink.

3. The use according to claim 2, wherein the agent is a food and the food is in a form of tablets, soft jellies, hard candies, soft candies, chocolates or chewing gums.

4. The use according to claim 2, wherein the agent is a drink and the drink is in a form of soft drinks, near water, sports drinks, jelly drinks, tea drinks, cocoa drinks, or coffee drinks.

5. The use according to claim 1, wherein the agent for sustaining concentration and attention is manufactured such that 5 to 50g of palatinose is taken at once.

## Patentansprüche

1. Die Verwendung von Palatinose in der Herstellung eines Wirkstoffs zur Aufrechterhaltung der mentalen Konzentrationsfähigkeit und Aufmerksamkeit.

2. Die Verwendung nach Anspruch 1, bei der es sich bei dem Wirkstoff zur Aufrechterhaltung der Konzentrationsfähigkeit und Aufmerksamkeit um eine Speise oder ein Getränk handelt.

3. Die Verwendung nach Anspruch 2, bei der der Wirkstoff eine Speise ist und die Speise in Form von Tabletten, Weingummi, Bonbons, Kaubonbons, Schokolade oder Kaugummi vorliegt.

4. Die Verwendung nach Anspruch 2, bei der der Wirkstoff ein Getränk ist und das Getränk in Form von Erfrischungsgetränken, Near-Water-Getränken, Sportgetränken, Jelly Drinks, Teegetränken, Kakaogetränken oder Kaffeegetränken vorliegt.

5. Die Verwendung nach Anspruch 1, bei der der Wirkstoff zur Aufrechterhaltung der Konzentrationsfähigkeit und Aufmerksamkeit so hergestellt wird, dass 5 bis 50g Palatinose auf einmal eingenommen werden.

## Revendications

1. L'utilisation de palatinose dans la fabrication d'un agent destiné à prolonger la concentration et l'attention mentales.

2. L'utilisation selon la revendication 1, dans laquelle l'agent destiné à prolonger la concentration et l'attention est un aliment ou une boisson.

3. L'utilisation selon la revendication 2, dans laquelle l'agent est un aliment et l'aliment se présente sous la forme de comprimés, de gelées, de bonbons durs, de bonbons tendres, de chocolats ou de chewing-gums.

4. L'utilisation selon la revendication 2, dans laquelle l'agent est une boisson et la boisson se présente sous la forme de boissons non alcoolisées, de boissons proches de l'eau, de boissons pour sportifs, de boissons gélifiées, de boissons à base de thé, de boissons cacaotées, ou de boissons à base de café.

5. L'utilisation selon la revendication 1, dans laquelle l'agent destiné à prolonger la concentration et l'attention est fabriqué de façon que 5 à 50 g de palatinose soient pris en une fois.
